(19)

Europäisches
Patentamt

European
Patent Office

Office européen
des brevets

(11) **EP 4 635 988 A1**

(12) **EUROPEAN PATENT APPLICATION**
published in accordance with Art. 153(4) EPC

(43) Date of publication:
**22.10.2025  Bulletin 2025/43**

(21) Application number: 23902641.2

(22) Date of filing: **11.12.2023**

(51) International Patent Classification (IPC):
*C07K 19/00* (2006.01)     *C12N 15/62* (2006.01)
*A61K 39/395* (2006.01)    *A61K 38/17* (2006.01)
*A61P 29/00* (2006.01)     *A61P 37/02* (2006.01)
*A61P 19/02* (2006.01)

(52) Cooperative Patent Classification (CPC):
A61K 38/17; A61K 39/395; A61P 19/02;
A61P 29/00; A61P 37/02; C07K 19/00;
C12N 15/62; C12N 15/85

(86) International application number:
**PCT/CN2023/137845**

(87) International publication number:
**WO 2024/125445 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  12.12.2022  CN 202211591278

(71) Applicant: **Jiangsu Kanion Pharmaceutical Co.,
Ltd.
Lianyungang, Jiangsu 222047 (CN)**

(72) Inventors:
• **XIAO, Wei
  Lianyungang, Jiangsu 222047 (CN)**
• **LIU, Zhihang
  Lianyungang, Jiangsu 222047 (CN)**
• **SONG, Liying
  Lianyungang, Jiangsu 222047 (CN)**

• **YIN, Chengkai
  Lianyungang, Jiangsu 222047 (CN)**
• **LIU, Tianyan
  Lianyungang, Jiangsu 222047 (CN)**
• **YU, Dan
  Lianyungang, Jiangsu 222047 (CN)**
• **LI, Deshan
  Lianyungang, Jiangsu 222047 (CN)**
• **WANG, Zhenzhong
  Lianyungang, Jiangsu 222047 (CN)**

(74) Representative: **Prinz & Partner mbB
Patent- und Rechtsanwälte
Leipziger Platz 15
10117 Berlin (DE)**

Remarks:
The complete document including Reference
Table(s) and the Sequence Listing(s) can be
downloaded from the EPO website

(54) **BISPECIFIC FUSION PROTEIN TARGETING TNF-? AND IL-17A, AND USE THEREOF**

(57)    The present invention relates to a bispecific fusion protein targeting TNF-α and IL-17A, a polynucleotide encoding same, a preparation method therefor, the use thereof, etc. The bispecific fusion protein targeting TNF-α and IL-17A is a dimer which has a bilaterally symmetrical structure, and comprises, in the order from N-terminal to C-terminal, three structural functional regions: a soluble TNF receptor or a portion thereof, a human IgG Fc fragment, and a functional domain competitively binding to IL-17A or against IL-17A. The fusion protein can effectively bind to both TNF-α and IL-17A, and has the effect of blocking the signal pathway thereof. The fusion protein has a good stability, specificity and biological activity.

EP 4 635 988 A1

**Description**

**TECHNICAL FIELD**

The present application belongs to the field of medical biotechnology. Specifically, the present application relates to a bispecific fusion protein targeting TNF-$\alpha$ and IL-17A, a polynucleotide encoding the same, a formulation and a pharmaceutical composition comprising the same, a preparation method thereof, and use thereof.

**BACKGROUND**

Tumor necrosis factor-$\alpha$ (TNF-$\alpha$) is an important inflammatory factor, which is mainly produced by cells such as macrophages, monocytes, T cells, NK cells, mast cells, and neutrophils. TNF-$\alpha$ is a transmembrane protein with a size of about 26 kDa that is exposed on a cell surface. It can be processed into an activated, soluble TNF-$\alpha$ with a size of about 17 kDa through proteolytic cleavage by TNF converting enzyme (TACE). Soluble TNF-$\alpha$ can form a trimer and can activate TNFR. TNF-$\alpha$ has two receptors, TNFR1 and TNFR2, and the functions mediated by the two receptors exhibit certain differences. TNFR1 of 55 kDa (p55) is expressed in all cells, whereas TNFR2 (p75) is mainly expressed in immune system cells and endothelial cells. The two receptors can also be cleaved from a cell membrane to form soluble forms. These soluble receptors can serve as antagonists, by binding to soluble TNF-$\alpha$, preventing TNF-$\alpha$ from binding to cell surface TNFRs.

In the synovium of joints of rheumatoid arthritis (RA) patients, activated macrophages and the like can produce TNF-$\alpha$, which can induce the synthesis of various other pro-inflammatory cytokines, chemokines, such as IL-1$\beta$, IL-6, and IL-8. These factors can reactivate macrophages at the joint, thereby making them produce cytokines continuously, causing abnormal proliferation of FLS (Fibroblast-like synovial cells), increasing the expression of adhesion factor, and inducing neovascularization. In addition, TNF-$\alpha$ can induce MMP synthesis, thereby resulting in the destruction of cartilage and bone tissue, and TNF-$\alpha$ can stimulate chondrocytes to secrete Wnt inhibitory factor DKK-1, inhibit the chondrogenesis in tissues and formation of bone tissue. As an important pro-inflammatory factor, TNF-$\alpha$ can also interact with various other cytokines in the occurrence and progression of RA, producing synergistic effects, increasing inflammation response *in vivo*. TNF-$\alpha$ can also inhibit the differentiation of Treg cells by inhibiting the transcription of Foxp3, reducing the content of Treg cells in the body, causing abnormal immune regulation, thereby aggravating the condition of RA. In recent years, the role of TNF-$\alpha$ in the pathology and physiology of RA has been deeply investigated. Moreover, the current biologics targeting TNF-$\alpha$ for treatment of rheumatoid arthritis (e.g., infliximab, adalimumab, golimumab, and certolizumab) have been widely used and have achieved good efficacy. Despite the obvious clinical benefits of anti-TNF-$\alpha$ agents, there are also problems such as non-response, secondary loss of response, and intolerance to anti-TNF agents.

Interleukin-17A (IL-17A) was first mentioned in 1995 as a pro-inflammatory cytokine produced by T cells. IL-17A, also commonly known as IL-17, is one of the six members of the IL-17 family. The subsequent studies found that Th17 cells are the main source of IL-17, and T cells are induced by IL-6, IL-1$\beta$, TGF-$\beta$ to produce Th17 cells which are matured by IL-23. Th17 cells are a class of highly pro-inflammatory T cells. IL-17A secreted by Th17 cells can have multiple effects on immune cells, can stimulate immune cells to produce various other inflammatory factors such as IL-1$\beta$, TNF-$\alpha$, IL-6, and CXC chemokines. Compared with healthy individuals, a large number of Th17 cells and IL-17A with higher concentrations can be found in the peripheral blood of RA patients. In addition, the synovial fluid in RA patients also contains a large number of Th17 cells. Studies have shown that Th17 cells secrete substantial IL-17A at synovial site, while the transcriptional level of IL-17A in synovial tissue is positively correlated with the progression of joint injury.

DMARDs (such as methotrexate) are still the mainstream drugs for the treatment of diseases such as rheumatoid arthritis. In recent years, a series of biologics, such as TNF-$\alpha$ inhibitors, have brought new hope to patients with poor treatment efficacy of DMARDs. However, clinical trials have shown that half of rheumatoid arthritis patients treated with TNF-$\alpha$ inhibitors fail to achieve satisfactory results. In addition, for some rheumatoid arthritis patients treated with single-target biologic drugs for a period of time, the efficacy of the drugs may gradually diminish over time, necessitating a switch to alternative medications.

In our previous study (see CN106892982B), we demonstrated that a fusion protein targeting both TNF-$\alpha$ and IL-17A antigens can achieve good efficacies *in vivo* and *in vitro;* but in the production process of fusion proteins and bispecific antibodies, we found that there are some main problems such as the production of aggregates, poor stability and low expression, so further research and exploration are needed.

Bispecific fusion proteins, like bispecific antibodies, have consistently faced challenges, including low expression levels, poor stability, complex process, and tendency to form aggregates. Therefore, there is a need to develop a bispecific fusion protein for the treatment of inflammation-related diseases with advantages such as better specificity, superior therapeutic efficacy and improved stability compared to existing fusion proteins.

## SUMMARY OF THE INVENTION

Through studies, the applicant develops a bispecific fusion protein targeting TNF-α and IL-17A that is distinct from the prior art, which is a dimer having a bilaterally symmetrical structure and comprising three functional domains, thereby providing a bispecific fusion protein directed to inflammatory factors that is structurally stable, highly specific and easy to prepare. The fusion protein can effectively bind to both TNF-α and IL-17A, and thus exerts the function of blocking the signal pathway thereof, exhibiting good biological activity.

In one aspect, the present application provides a bispecific fusion protein targeting TNF-α and IL-17A, comprising, in the order from N-terminal to C-terminal:

a soluble TNF receptor or a portion thereof;
a human IgG Fc fragment; and
a functional domain competitively binding to IL-17A or against IL-17A.

In another aspect, the present application provides a polynucleotide encoding the above bispecific fusion protein.

In another aspect, the present application provides a recombinant vector comprising the above polynucleotide.

In another aspect, the present application provides a host cell comprising the above polynucleotide or recombinant vector.

In another aspect, the present application provides a method of preparing the bispecific fusion protein described herein, comprising:

a) preparing a polynucleotide encoding the above bispecific fusion protein;
b) constructing a recombinant vector by using the polynucleotide and an expression vector;
c) transferring the recombinant vector into host cells and culturing transformed cells to obtain cell culture; and
d) purifying and isolating the cell culture to obtain the bispecific fusion protein.

In another aspect, the present application provides use of the above bispecific fusion protein in the preparation of a medicament for treating an inflammation-related disease. Alternatively, the present application provides use of the above bispecific fusion protein in the preparation of a reagent for binding and inhibiting TNF-α and IL-17A.

In another aspect, the present application provides a formulation comprising the above bispecific fusion protein, wherein the formulation further comprises a buffer system, a pharmaceutically acceptable excipient and a surfactant, in which the buffer system is selected from a solution of acetic acid and sodium acetate, a solution of citric acid and sodium citrate, or a solution of histidine and histidine hydrochloride.

In another aspect, the present application provides a pharmaceutical composition comprising the above bispecific fusion protein.

Advantageous Effects

The bispecific fusion protein targeting TNF-α and IL-17A in the present application is a dimer which has a bilaterally symmetrical structure and comprises three structural functional domains. The fusion protein can effectively bind to both TNF-α and IL-17A, and exerts the function of blocking the signal pathway thereof, particularly inhibiting the synergistic effects caused by these two signal pathways. The fusion protein has a good stability, specificity and biological activity, which can be used in individual treatment, prevention and/or diagnosis of related diseases.

Compared with the prior art (e.g., CN 109796534A), the present application improves protein stability and reduces the formation of aggregates by structural optimization of the fusion protein, while exhibiting good affinity and other effects. Furthermore, it makes the fusion protein more stable, produces fewer aggregates and has better druggability in the formulation by exploring the formula of the formulation.

## DETAILED DESCRIPTION OF THE DRAWINGS

Figure 1 shows structures of eight exemplary proteins designed in Example 1 of the present application.

Figure 2 shows SDS-PAGE analysis results of eight exemplary purified proteins designed in Example 1 of the present application.

Figure 3 shows SEC-HPLC analysis results of eight exemplary purified proteins designed in Example 1 of the present application.

Figure 4 shows binding response values of each of eight exemplary proteins designed in Example 1 of the present application to two antigens, wherein the binding ratio of each protein to the two antigens should theoretically be 1:2.

Figure 5 shows reduced and non-reduced SDS-PAGE analyses of fusion proteins of Nos. 5, 6, and 8 before storage and after storage at -80°C.

Figure 6 is a schematic diagram of the structure of No. 5 protein.

Figure 7 shows information of purified No. 5 protein through CHO transient expression.

Figure 8 shows osteoarticular scores of mice after treatment for hIL-17A transgenic rheumatoid arthritis in each group.

Figure 9 shows images of paws of mice in each group after treatment.

Figure 10 shows MicroCT results of paws (front left, front right, rear left, rear right) of representative mice in each group.

Figure 11 shows MicroCT bone injury scores of mice in each group (3 individuals scored back-to-back, taking the average).

## EMBODIMENTS OF THE INVENTION

Hereinafter, the present application is further described in combination with particular embodiments. It should be understood by the person skilled in the art that these embodiments are merely exemplary and do not limit the scope of the present application hereto. The person skilled in the art may make various modifications, alterations, substitutions, or combinations to the technical solutions of the present application without departing from the spirit and scope thereof, and the solutions thus obtained fall within the scope of protection of the present application.

Unless defined otherwise, the technical and scientific terms used herein have the same meanings as commonly understood by the person of ordinary skill in the art to which this disclosure belongs, which can be seen in, for example, Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989).

Unless otherwise specified, the term "vector" herein refers to a self-replicating DNA molecule used to transfer a target gene (e.g., an exogenous gene from the same or different species) into a recipient cell.

Unless otherwise specified, the term "linker peptide" herein may refer to a peptide sequence used to link each component within the fusion protein herein together.

Unless otherwise specified, the terms "polynucleotide", "nucleic acid", "nucleotide sequence", and "gene" herein are used interchangeably, all of which are chemical substances in essence.

Unless otherwise specified, the term "treatment" herein means curing, reducing, alleviating, slowing down, palliating or ameliorating a disease or related symptoms in a statistically significant manner, or preventing, delaying, stopping, discontinuing or halting the onset or further progression of a disease or related symptoms.

As used herein, the term "antibody" refers to a binding protein having at least one antigen-binding domain. The antibody and antigen-binding fragment thereof in the present application can be an intact antibody or any antigen-binding fragment thereof. Thus, the antibody and antigen-binding fragment thereof in the present application comprise a monoclonal antibody or antigen-binding fragment thereof, an antibody variant or antigen-binding fragment thereof, and an immunoconjugate. Examples of antibody fragments comprise Fab fragments, Fab' fragments, F(ab')2 fragments, Fv fragments, isolated CDR regions, single-chain Fv molecules (scFv), single-domain antibodies (sdAb), Fd fragments, and other antigen-binding fragments known in the art.

The term "pharmaceutically acceptable" refers to compounds, materials, compositions and/or dosage forms that are, within the scope of sound medical judgment, suitable for contact with human and animal tissues without excessive toxicity, irritation, allergic reactions or other problems or complications, and are commensurate with a reasonable benefit/risk ratio.

Unless otherwise specified, the terms "comprise", "comprises", and "comprising", or their equivalents herein are open-ended expressions, which means that in addition to the listed elements, components and steps, other unspecified elements, components and steps may also be covered.

The identity percentage (degree of homology) between sequences herein can be determined by aligning two or more sequences using, for example, a freely available computer program commonly used for such purpose on the World Wide Web such as BLASTp or BLASTn with default settings.

Unless otherwise specified, all the numbers used herein to express amounts of ingredients, measured values, or test conditions should be understood as being modified in all cases by the term "about". When being connected to a percentage, the term "about" can represent, for example, ±1%, preferably ±0.5%, more preferably ±0.1%. Unless clearly indicated otherwise, a singular term herein encompasses plural referents and *vice versa*. Unless clearly indicated otherwise, the word "or" herein intends to include "and".

In one embodiment, the present application provides a bispecific fusion protein, comprising, in the order from N-terminal to C-terminal:

a soluble TNF receptor or a portion thereof;
a human IgG Fc fragment; and
a functional domain competitively binding to IL-17A or against IL-17A.

In some embodiments, the soluble TNF receptor or a portion thereof can be soluble TNF receptor 1 (abbreviated as sTNFRI or sTNFR1) or a portion thereof, or soluble TNF receptor 2 (abbreviated as sTNFRII or sTNFR2) or a portion thereof. In some preferred embodiments, in terms of achieving higher expression and superior affinity for target proteins, the soluble TNF receptor or a portion thereof can preferably be soluble TNF receptor 1 or a portion thereof. In more preferred embodiments, the soluble TNF receptor or the portion thereof can be an extracellular fragment of soluble TNF receptor 1.

In some embodiments, the extracellular fragment of soluble TNF receptor 1 comprises an amino acid sequence set forth in SEQ ID NO: 1 or an amino acid sequence having at least 95%, 96%, 97%, 98%, 99%, or 100% identity thereto, or a conservatively modified variant thereof (e.g., insertion, deletion, substitution, etc.):

REKRDSVCPQGKYIHPQNNSICCTKCHKGTYLYNDCPGPGQDTDCRECESGSFTASENHLR
HCLSCSKCRKEMGQVEISSCTVDRDTVCGCRKNQYRHYWSENLFQCFNCSLCLNGTVHLS
CQEKQNTVCTCHAGFFLRENECVSCSNCKKSLECTKLCL (SEQ ID NO: 1).

And, the extracellular fragment of soluble TNF receptor 1 or variant thereof described herein retains the ability to specifically recognize and bind to TNFα.

Conservative modification herein refers to amino acid modifications that do not significantly affect or change antibody binding characteristics. For example, a conservative amino acid substitution can refer to the substitution with another amino acid of the same class (having similar chemical properties or functions). As an example, according to their side-chain properties, amino acids can be grouped into: (1) nonpolar amino acids: Ala (A), Val (V), Leu (L), Ile (I), Pro (P), Phe (F), Trp (W), Met (M); (2) uncharged polar amino acids: Gly (G), Ser (S), Thr (T), Cys (C), Tyr (Y), Asn (N), Gln (Q); (3) acidic amino acids: Asp (D), Glu (E); (4) basic amino acids: Lys (K), Arg (R), His (H). Alternatively, based on the common side-chain properties, amino acids can be grouped into: (1) hydrophobic amino acids: Met, Ala, Val, Leu, Ile; (2) neutral hydrophilic amino acids: Cys, Ser, Thr, Asn, Gln; (3) acidic amino acids: Asp, Glu; (4) basic amino acids: His, Lys, Arg; (5) amino acids affecting chain orientation: Gly, Pro; (6) aromatic amino acids: Trp, Tyr, Phe.

A human IgG Fc fragment is included in the fusion protein herein to prolong the half-life thereof. In some embodiments, the human IgG Fc fragment is a human IgG1 Fc or IgG4 Fc fragment.

In some embodiments, the human IgG Fc fragment is a Hinge-CH2-CH3 fragment of IgG4, more preferably a human IgG4 Fc fragment with S228P mutation.

In some embodiments, the IgG4 Fc fragment comprises an amino acid sequence set forth in SEQ ID NO: 2 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity thereto:

ESKYGPPCPPCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK (SEQ ID NO: 2).

In some embodiments, the functional domain competitively binding to IL-17A or against IL-17A can be selected from: a receptor of IL-17A, a variant thereof or a portion thereof, or an anti-IL-17A antibody or an antigen-binding fragment thereof.

In some embodiments, the antigen-binding fragment of the anti-IL-17A antibody includes, but is not limited to, a Fab fragment, a Fab' fragment, a F(ab')2 fragment, an Fv fragment, a single-chain fragment variable (scFv), a single-domain antibody (sdAb), an isolated CDR region, or an Fd fragment from the antibody.

In some embodiments, the functional domain competitively binding to IL-17A or against IL-17A can be an anti-IL-17A single-chain fragment variable.

In some embodiments, the anti-IL-17A single-chain fragment variable comprises:

a heavy chain CDR1 (HCDR1) comprising an amino acid sequence set forth in SEQ ID NO: 5;
a heavy chain CDR2 (HCDR2) comprising an amino acid sequence set forth in SEQ ID NO: 6;
a heavy chain CDR3 (HCDR3) comprising an amino acid sequence set forth in SEQ ID NO: 7;
a light chain CDR1 (LCDR1) comprising an amino acid sequence set forth in SEQ ID NO: 8;
a light chain CDR2 (LCDR2) comprising an amino acid sequence set forth in SEQ ID NO: 9;
a light chain CDR3 (LCDR3) comprising an amino acid sequence set forth in SEQ ID NO: 10.

In some embodiments, the anti-IL-17A single-chain fragment variable comprises: a heavy chain CDR1 (HCDR1) set forth in SEQ ID NO: 5, a heavy chain CDR2 (HCDR2) set forth in SEQ ID NO: 6 and a heavy chain CDR3 (HCDR3) set forth in SEQ ID NO: 7, as well as a light chain CDR1 (LCDR1) set forth in SEQ ID NO: 8, a light chain CDR2 (LCDR2) set forth in SEQ ID NO: 9 and a light chain CDR3 (LCDR3) set forth in SEQ ID NO: 10:

GYSFTDYHIH (SEQ ID NO: 5);
VINPMYGTTDYNQRFKG (SEQ ID NO: 6);
YDYFTGTGVY (SEQ ID NO: 7);
RSSRSLVHSRGNTYLH (SEQ ID NO: 8);
KVSNRFI (SEQ ID NO: 9);
SQSTHLPFT (SEQ ID NO: 10).

The ranges of the above CDRs have been defined according to the Kabat numbering scheme (see Kabat, et al., Ann. NY Acad. Sci. 190:382-93 (1971); Kabat, et al., Sequences of Proteins of Immunological Interest, Fifth Edition, U.S. Department of Health and Human Services, NIH Publication No. 91-3242 (1991)), but it will be understood by the person skilled in the art that CDR sequences numbered according to any one or more numbering schemes such as the Chothia numbering scheme, the ImMunoGenTics (IMGT) numbering scheme, the AbM numbering scheme and the Contact numbering scheme (based on an analysis of available complex crystal structures) also fall within the protection scope of the present application.

In some embodiments, the anti-IL-17A single-chain fragment variable comprises a heavy chain variable region comprising an amino acid sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity thereto, and a light chain variable region comprising an amino acid sequence set forth in SEQ ID NO: 12 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity thereto:

QVQLVQSGAEVKKPGSSVKVSCKASGYSFTDYHIHWVRQAPGQGLEWMGVINPMYGTTD
YNQRFKGRVTITADESTSTAYMELSSLRSEDTAVYYCARYDYFTGTGVYWGQGTLVTVSS
(SEQ ID NO: 11);

DIVMTQTPLSLSVTPGQPASISCRSSRSLVHSRGNTYLHWYLQKPGQSPQLLIYKVSNRFIG
VPDRFSGSGSGTDFTLKISRVEAEDVGVYYCSQSTHLPFTFGQGTKLEIKRT (SEQ ID NO: 12).

In some embodiments, the anti-IL-17A single-chain fragment variable comprises an amino acid sequence set forth in SEQ ID NO: 3 or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity thereto:

QVQLVQSGAEVKKPGSSVKVSCKASGYSFTDYHIHWVRQAPGQGLEWMGVINPMYGTTD
YNQRFKGRVTITADESTSTAYMELSSLRSEDTAVYYCARYDYFTGTGVYWGQGTLVTVSS
GGGGSGGGGSGGGGSDIVMTQTPLSLSVTPGQPASISCRSSRSLVHSRGNTYLHWYLQKPG
QSPQLLIYKVSNRFIGVPDRFSGSGSGTDFTLKISRVEAEDVGVYYCSQSTHLPFTFGQGTKL
EIKRT (SEQ ID NO: 3).

Furthermore, the variant of the anti-IL-17A single-chain fragment variable herein retains the ability to specifically recognize and bind to IL-17A.

In some embodiments, the bispecific fusion protein described herein comprises, in the order from N-terminal to C-terminal: (1) soluble TNF receptor 1 or 2, or an extracellular fragment thereof, (2) a human IgG4 Fc fragment, and (3) an anti-IL-17A single-chain fragment variable.

In a preferred embodiment, the bispecific fusion protein described herein comprises, in the order from N-terminal to C-terminal: (1) soluble TNF receptor 1 or an extracellular fragment thereof, (2) a human IgG4 Fc fragment, and (3) an anti-IL-17A single-chain fragment variable.

In a preferred embodiment, the bispecific fusion protein described herein comprises, in the order from N-terminal to C-terminal: (1) an extracellular fragment of soluble TNF receptor 1, (2) a human IgG4 Fc fragment, and (3) an anti-IL-17A single-chain fragment variable comprising a heavy chain CDR1 (HCDR1) set forth in SEQ ID NO: 5, a heavy chain CDR2 (HCDR2) set forth in SEQ ID NO: 6 and a heavy chain CDR3 (HCDR3) set forth in SEQ ID NO: 7, as well as a light chain CDR1 (LCDR1) set forth in SEQ ID NO: 8, a light chain CDR2 (LCDR2) set forth in SEQ ID NO: 9 and a light chain CDR3 (LCDR3) set forth in SEQ ID NO: 10.

In a preferred embodiment, the bispecific fusion protein described herein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising a heavy chain CDR1 (HCDR1) set forth in SEQ ID NO: 5, a heavy chain CDR2 (HCDR2) set forth in SEQ ID NO: 6 and a heavy chain CDR3 (HCDR3) set forth in SEQ ID NO: 7, as well as a light chain CDR1 (LCDR1) set forth in SEQ ID NO: 8, a light chain CDR2 (LCDR2) set forth in SEQ ID NO: 9 and a light chain CDR3 (LCDR3) set forth in SEQ ID NO: 10.

In a preferred embodiment, the bispecific fusion protein described herein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising a heavy chain variable region comprising an amino acid sequence set forth in SEQ ID NO: 11 and a light chain variable region comprising an amino acid sequence set forth in SEQ ID NO: 12.

In a preferred embodiment, the bispecific fusion protein described herein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable set forth in SEQ ID NO: 3.

In some embodiments, the human IgG Fc fragment is linked to the functional domain competitively binding to IL-17A or against IL-17A via a linker peptide.

In some embodiments, the linker peptide can be a flexible linker peptide or a rigid linker peptide. In some embodiments, the linker peptide is a GS flexible linker peptide (e.g., a flexible peptide containing GSG repeating units, a flexible peptide containing G4S repeating units, or a flexible peptide containing the two), more preferably (G4S)4 (i.e., GGGGSGGGGSGGGGSGGGGS (SEQ ID NO: 4)).

In some embodiments, the bispecific fusion protein comprises, in the order from N-terminal to C-terminal: soluble TNF receptor 1 or an extracellular fragment thereof, a human IgG4 Fc fragment, a GS flexible linker peptide and an anti-IL-17A single-chain fragment variable.

In a preferred embodiment, the bispecific fusion protein described herein comprises, in the order from N-terminal to C-terminal: an extracellular fragment of soluble TNF receptor 1, a human IgG4 Fc fragment, a GS flexible linker peptide, and an anti-IL-17A single-chain fragment variable comprising a heavy chain CDR1 (HCDR1) set forth in SEQ ID NO: 5, a heavy chain CDR2 (HCDR2) set forth in SEQ ID NO: 6 and a heavy chain CDR3 (HCDR3) set forth in SEQ ID NO: 7, as well as a light chain CDR1 (LCDR1) set forth in SEQ ID NO: 8, a light chain CDR2 (LCDR2) set forth in SEQ ID NO: 9 and a light chain CDR3 (LCDR3) set forth in SEQ ID NO: 10.

In a preferred embodiment, the bispecific fusion protein described herein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a heavy chain CDR1 (HCDR1) set forth in SEQ ID NO: 5, a heavy chain CDR2 (HCDR2) set forth in SEQ ID NO: 6 and a heavy chain CDR3 (HCDR3) set forth in SEQ ID NO: 7, as well as a light chain CDR1 (LCDR1) set forth in SEQ ID NO: 8, a light chain CDR2 (LCDR2) set forth in SEQ ID NO: 9 and a light chain CDR3 (LCDR3) set forth in SEQ ID NO: 10.

In a preferred embodiment, the bispecific fusion protein described herein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a heavy chain variable region comprising an amino acid sequence set forth in SEQ ID NO: 11 and a light chain variable region comprising an amino acid sequence set forth in SEQ ID NO: 12.

Preferably, the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable set forth in SEQ ID NO: 3. The bispecific fusion protein can specifically recognize and bind to IL-17A and TNF$\alpha$.

In some preferred embodiments, the polypeptide chain of the fusion protein forms a homodimer through disulfide bonds between the Fc fragments therein. Herein, the soluble TNF receptor or a portion thereof is linked to the hinge region of the IgG Fc fragment, wherein the function of the soluble TNF receptor or a portion thereof is ensured through the flexibility of the hinge region, and through the disulfide bonds generated in the hinge region, the fusion protein is ensured to form a dimer.

In some preferred embodiments, the fusion protein is a bilaterally symmetrical dimer.

In some embodiments, the fusion protein described in the present application has an affinity constant (KD) of $9\times10^{-12}$ M or less for IL-17A, preferably $7\times10^{-12}$ M or less, more preferably $6.5\times10^{-12}$ M or less; and has an affinity constant (KD) of $4\times10^{-11}$ M or less for TNF$\alpha$, preferably $3.8\times10^{-11}$ M or less, more preferably $3.6\times10^{-11}$ M or less.

The fusion protein of the present application can be formulated into any dosage form suitable for being administered to a subject, for example, the fusion protein can be administered intravenously, intramuscularly, parenterally, orally, subcutaneously, intrathecally, intraventricularly, intraspinally, intraperitoneally, intrauterinely, intranasally, etc. In some preferred embodiments, the fusion protein is in a form of lyophilized powder or injection.

In one embodiment, the present application provides a polynucleotide which can encode the above bispecific fusion protein.

In one embodiment, the present application provides a recombinant vector comprising a polynucleotide which can encode the fusion protein.

Herein, any expression vector known in the art can be used to construct the recombinant vector with the above polynucleotide encoding the fusion protein. The expression vector can employ, such as a pcDNA series vector (pcDNA3.1 vector, pcDNA3.2 vector, pcDNA3.3 vector, pcDNA3.4 vector (e.g., pcDNA3.4-TOPO TA vector)), pBK-CMV vector, pEGFP-N1 vector, and pGenHT 1.0-DGV vector.

In one embodiment, the present application provides a host cell comprising the above polynucleotide or recombinant vector, thereby expressing the bispecific fusion protein described in the present application.

In a preferred embodiment, the host cell expressing the fusion protein is a prokaryotic or eukaryotic cell, more preferably a mammalian cell, such as HEK293 cells (e.g., 293T cells, 293F cells, 293H cells or 293S cells), CHO cells (e.g., CHOK1-GenS cells), BHK cells, and Sp2/0 cells.

The above polynucleotide or recombinant vector can be transferred into the host cell by any suitable technique known in the art for transferring exogenous genes into host cells (e.g., electroporation, transduction, transfection), thereby the resulting host cells can efficiently express the bispecific fusion protein in the present application.

Herein, the culture of cells into which exogenous genes have been transferred can be carried out by the person skilled in the art via selecting conventional culture medium and culture conditions based on the types of cells ("Cell Culture (3rd Edition)", Bin LIU, Editor in Chief, World Publishing Corporation, January 2018; "Cell Culture Technology", Rong LAN and Zhenhui ZHOU, Editor in Chief, Chemical Industry Press, August 2007; "Tissue and Cell Culture Technology (3rd Edition)", Jingbo ZHANG, Editor in Chief, People's Medical Publishing House, June 2014, etc.).

In one embodiment, the present application provides a method of preparing the bispecific fusion protein, comprising:

a) preparing a polynucleotide encoding the above bispecific fusion protein;
b) constructing a recombinant vector by using the polynucleotide and an expression vector;
c) transferring the recombinant vector into host cells and culturing transformed cells to obtain cell culture;
d) purifying and isolating the cell culture to obtain the bispecific fusion protein.

Herein, the polynucleotide can be synthesized according to the amino acid sequence of the corresponding fusion protein by techniques such as conventional whole gene synthesis methods or enzymatic synthesis.

Herein, the recombinant vector can be constructed from the polynucleotide and the expression vector through conventional enzyme cleavage and ligation.

As described above, the transformed cells can be cultured by the person skilled in the art via selecting conventional culture medium and culture conditions based on the types of cells. In some embodiments, the cells are cultured by a fed-batch culture mode or a perfusion culture mode, more preferably, the cells are cultured by a high-density fed-batch culture mode or a perfusion culture mode.

Herein, the obtained cell culture can be purified by conventional protein purification methods, such as but not limited to electrophoresis, ultracentrifugation, dialysis, ultrafiltration, precipitation, chromatography (e.g., gel filtration chromatography (such as size exclusion chromatography (SEC)), ion exchange chromatography, affinity chromatography, hydrophobic interaction chromatography, reversed-phase chromatography, high-performance liquid chromatography and multimodal chromatography). In some preferred embodiments, the purification is performed using affinity chromatography and multimodal chromatography; for example, the purification can be performed by Protein A affinity column. In further preferred embodiments, aggregates of the fusion protein are removed using a hydroxyapatite (CHT) composite filler.

In one embodiment, the present application provides a formulation comprising the above bispecific fusion protein, wherein the formulation further comprises a buffer system, a pharmaceutically acceptable excipient and a surfactant, in which the buffer system is selected from a solution of acetic acid and sodium acetate, a solution of citric acid and sodium citrate, or a solution of histidine and histidine hydrochloride.

In some embodiments, the formulation has a pH value of 3.0-7.5, preferably has a pH value of 3.5-6.5, more preferably has a pH value of 4.0-5.0.

In some embodiments, the formulation comprises: 5 mg/mL-100 mg/mL of the bispecific fusion protein, 5 mM-50 mM (e.g., 5 mM, 10 mM, 15 mM, 20 mM, 25 mM, 30 mM, 40 mM or 50 mM) of the buffer system, 50 mg/mL-100 mg/mL of the pharmaceutically acceptable excipient and 0.01%-0.05% (w/v) of the surfactant.

In some embodiments, the buffer system is selected from a 5 mM-20 mM (e.g., 10 mM) solution of acetic acid and sodium acetate, a 5 mM-20 mM (e.g., 10 mM) solution of citric acid and sodium citrate, and a 5 mM-20 mM (e.g., 10 mM) solution of histidine and histidine hydrochloride, more preferably, a 10 mM solution of acetic acid and sodium acetate.

In some embodiments, the pharmaceutically acceptable excipient is at least one of sucrose or trehalose, more preferably trehalose.

In some embodiments, the surfactant is a non-ionic surfactant, such as Span surfactants, polysorbate surfactants (e.g., polysorbate-20, polysorbate-60, polysorbate-80) and so on.

In one embodiment, the present application provides a pharmaceutical composition comprising the above bispecific fusion protein.

In preferred embodiments, the pharmaceutical composition further comprises a pharmaceutically acceptable excipient. The pharmaceutically acceptable excipient can be selected from, such as but not limited to, solvent, propellant, solubilizer, cosolvent, emulsifier, colorant, disintegrant, filler, lubricant, wetting agent, osmotic pressure regulator, stabilizer, glidant, flavoring agent, preservative, suspending agent, antioxidant, penetration enhancer, pH regulator, surfactant, diluent, etc. For other available pharmaceutically acceptable pharmaceutical excipients, they can be found, for example, in "Handbook of Pharmaceutical Excipients" (4th Edition), edited by R.C. Rowe et al., translated by Junmin ZHENG, 2005, Chemical Industry Press.

In one embodiment, the present application provides use of the above bispecific fusion protein, or the above formulation or pharmaceutical composition comprising the same, in the preparation of a medicament for treating an inflammation-related disease. Alternatively, the present application provides use of the above bispecific fusion protein, or the above formulation or pharmaceutical composition comprising the same, in the preparation of a reagent for binding and inhibiting TNF-α and IL-17A.

In some preferred embodiments, the reagent is used to block TNF and IL-17 signaling pathways, including inhibition of synergistic effects caused by these two pathways.

In another preferred embodiment, the reagent is used to block interactions between TNF and TNFR and/or IL-17 and IL-17 receptor complexes.

In one embodiment, the present application relates to a method of treating an inflammation-related disease, comprising administering to a subject in need thereof the above bispecific fusion protein, or the above formulation or pharmaceutical composition comprising the same. Alternatively, the present application provides the above bispecific fusion protein, formulation or pharmaceutical composition for use in treating an inflammation-related disease. Alternatively, the present application provides a method of binding and inhibiting TNF-α and IL-17A, comprising administering to a subject in need thereof the above bispecific fusion protein, or the above formulation or pharmaceutical composition comprising the same. Alternatively, the present application provides the above bispecific fusion protein, or the above formulation or pharmaceutical composition comprising the same, for use as a reagent for binding and inhibiting TNF-α and IL-17A.

In some embodiments, the subject is a mammal. In a preferred embodiment, the mammal is human.

In some embodiments, the inflammation-related disease is an autoimmune disease or a cytokine release syndrome, more preferably, is rheumatoid arthritis, psoriasis, psoriatic arthritis, ankylosing spondylitis, ulcerative colitis, a cytokine release syndrome caused by viral infections or immunomodulatory drugs.

The dosage, frequency and route of administration of the above bispecific fusion protein, or the above formulation or pharmaceutical composition comprising the same herein, can be determined by clinicians according to the patient's body weight, gender, age, condition, physical health status, etc.

Exemplary technical solutions of the present application can be explained through the content in the following numbered paragraphs:

1. A bispecific fusion protein, comprising, in the order from N-terminal to C-terminal:

a soluble TNF receptor or a portion thereof;
a human IgG Fc fragment; and
a functional domain competitively binding to IL-17A or against IL-17A.

2. The bispecific fusion protein of paragraph 1, wherein the soluble TNF receptor or the portion thereof is soluble TNF receptor 1 or a portion thereof, or soluble TNF receptor 2 or a portion thereof.

3. The bispecific fusion protein of paragraph 2, wherein the soluble TNF receptor or the portion thereof is soluble TNF receptor 1 or a portion thereof.

4. The bispecific fusion protein of any one of paragraphs 1-3, wherein the soluble TNF receptor or the portion thereof is an extracellular fragment of soluble TNF receptor 1.

5. The bispecific fusion protein of paragraph 4, wherein the extracellular fragment of soluble TNF receptor 1 comprises an amino acid sequence set forth in SEQ ID NO: 1 or an amino acid sequence having at least 95% identity thereto, or a conservatively modified variant thereof.

6. The bispecific fusion protein of any one of paragraphs 1-5, wherein the human IgG Fc fragment is a human IgG1 Fc or a human IgG4 Fc fragment.

7. The bispecific fusion protein of any one of paragraphs 1-6, wherein the human IgG Fc fragment is a Hinge-CH2-CH3 fragment of IgG4.

8. The bispecific fusion protein of any one of paragraphs 1-7, wherein the human IgG Fc fragment is a human IgG4 Fc fragment with S228P mutation.

9. The bispecific fusion protein of any one of paragraphs 1-8, wherein the functional domain competitively binding to IL-17A or against IL-17A is selected from: a receptor of IL-17A, a variant thereof or a portion thereof, or an anti-IL-17A antibody or an antigen-binding fragment thereof.

10. The bispecific fusion protein of paragraph 9, wherein the antigen-binding fragment of the anti-IL-17A antibody comprises a Fab fragment, a Fab' fragment, a F(ab')2 fragment, an Fv fragment, a single-chain fragment variable, a single-domain antibody, an isolated CDR region or an Fd fragment of the anti-IL-17A antibody.

11. The bispecific fusion protein of any one of paragraphs 1-9, wherein the functional domain competitively binding to IL-17A or against IL-17A is an anti-IL-17A single-chain fragment variable.

12. The bispecific fusion protein of paragraph 11, wherein the anti-IL-17A single-chain fragment variable comprises: a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10.

13. The bispecific fusion protein of paragraph 11 or 12, wherein the anti-IL-17A single-chain fragment variable comprises: a heavy chain variable region comprising an amino acid sequence set forth in SEQ ID NO: 11 or an amino acid sequence having at least 80% identity thereto, and a light chain variable region comprising an amino acid sequence set forth in SEQ ID NO: 12 or an amino acid sequence having at least 80% identity thereto.

14. The bispecific fusion protein of any one of paragraphs 11-13, wherein the anti-IL-17A single-chain fragment variable comprises an amino acid sequence set forth in SEQ ID NO: 3 or an amino acid sequence having at least 80% identity thereto.

15. The bispecific fusion protein of paragraph 1 or 2, wherein the bispecific fusion protein comprises, in the order from N-terminal to C-terminal: (1) soluble TNF receptor 1 or 2, or an extracellular fragment thereof, (2) a human IgG4 Fc fragment, and (3) an anti-IL-17A single-chain fragment variable.

16. The bispecific fusion protein of paragraph 15, wherein the bispecific fusion protein comprises, in the order from N-terminal to C-terminal: (1) soluble TNF receptor 1 or an extracellular fragment thereof, (2) a human IgG4 Fc fragment, and (3) an anti-IL-17A single-chain fragment variable.

17. The bispecific fusion protein of paragraph 16, wherein the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10.

18. The bispecific fusion protein of paragraph 17, wherein the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising: a heavy chain variable region comprising an amino acid sequence set forth in SEQ ID NO: 11, and a light chain variable region comprising an amino acid sequence set forth in SEQ ID NO: 12.

19. The bispecific fusion protein of paragraph 18, wherein the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable set forth in SEQ ID NO: 3.

20. The bispecific fusion protein of any one of paragraphs 1-9, wherein the human IgG Fc fragment is linked to the functional domain competitively binding to IL-17A or against IL-17A via a linker peptide.

21. The bispecific fusion protein of paragraph 20, wherein the linker peptide is a GS flexible linker peptide.

22. The bispecific fusion protein of any one of paragraphs 1-3, wherein the bispecific fusion protein comprises, in the order from N-terminal to C-terminal: soluble TNF receptor 1 or an extracellular fragment thereof, a human IgG4 Fc fragment, a GS flexible linker peptide and an anti-IL-17A single-chain fragment variable.

23. The bispecific fusion protein of paragraph 22, wherein the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10.

24. The bispecific fusion protein of paragraph 23, wherein the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a heavy chain variable region comprising an amino acid sequence set forth in SEQ ID NO: 11 and a light chain variable region comprising an amino acid sequence set forth in SEQ ID NO: 12.

25. The bispecific fusion protein of paragraph 24, wherein the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4 and an anti-IL-17A single-chain fragment variable set forth in SEQ ID NO: 3.

26. The bispecific fusion protein of any one of paragraphs 1-25, wherein the fusion protein has an affinity constant KD of $9 \times 10^{-12}$ M or less for IL-17A and an affinity constant KD of $4 \times 10^{-11}$ M or less for TNFα.

27. A polynucleotide, encoding the bispecific fusion protein of any one of paragraphs 1-26.

28. A recombinant vector, comprising the polynucleotide of paragraph 27.

29. A host cell, comprising the polynucleotide of paragraph 27 or the recombinant vector of paragraph 28.

30. A method of preparing the bispecific fusion protein of any one of paragraphs 1-26, comprising:

a) preparing a polynucleotide encoding the bispecific fusion protein;
b) constructing a recombinant vector by using the polynucleotide and an expression vector;
c) transferring the recombinant vector into host cells and culturing transformed cells to obtain cell culture; and
d) purifying and isolating the cell culture to obtain the bispecific fusion protein.

31. A formulation comprising the bispecific fusion protein of any one of paragraphs 1-26, wherein the formulation further comprises a buffer system, a pharmaceutically acceptable excipient, and a surfactant, in which the buffer system is selected from a solution of acetic acid and sodium acetate, a solution of citric acid and sodium citrate, or a solution of histidine and histidine hydrochloride.

32. The formulation of paragraph 31, wherein the formulation has a pH value of 3.0-7.5.

33. The formulation of paragraph 31 or 32, wherein the formulation comprises: 5 mg/mL-100 mg/mL of the bispecific fusion protein, 5 mM-50 mM of the buffer system, 50 mg/mL-100 mg/mL of the pharmaceutically acceptable excipient and 0.01%-0.05% (w/v) of the surfactant.

34. The formulation of any one of paragraphs 31-33, wherein the buffer system is selected from a 5 mM-20 mM solution of acetic acid and sodium acetate, a 5 mM-20 mM solution of citric acid and sodium citrate, or a 5 mM-20 mM solution of histidine and histidine hydrochloride.

35. The formulation of any one of paragraphs 31-34, wherein the pharmaceutically acceptable excipient is at least one of sucrose or trehalose.

36. The formulation of any one of paragraphs 31-35, wherein the surfactant is a non-ionic surfactant.

37. A pharmaceutical composition, comprising the bispecific fusion protein of any one of paragraphs 1-26.

38. Use of the bispecific fusion protein of any one of paragraphs 1-26, the formulation of any one of paragraphs 31-36 or the pharmaceutical composition of paragraph 37 in the preparation of a medicament for treating an inflammation-related disease.

39. The use of paragraph 38, wherein the inflammation-related disease is an autoimmune disease or a cytokine release syndrome.

40. The use of paragraph 39, wherein the inflammation-related disease is rheumatoid arthritis, psoriasis, psoriatic arthritis, ankylosing spondylitis, ulcerative colitis, a cytokine release syndrome caused by viral infections or immunomodulatory drugs.

41. Use of the bispecific fusion protein of any one of paragraphs 1-26, the formulation of any one of paragraphs 31-36 or the pharmaceutical composition of paragraph 37 in the preparation of a reagent for binding and inhibiting TNF-$\alpha$ and IL-17A.

For the purposes of description and disclosure, all patents, patent applications, and other identified publications are hereby expressly incorporated by reference. These publications are provided only because their disclosures are earlier than the filing date of the present application. All statements regarding the dates of these documents or expressions regarding the contents of these documents are based on information available to the applicant, and do not constitute any admission of the correctness of the date of these documents or the contents of these documents. Besides, in any country, any reference to these publications herein does not constitute an admission that the publication is part of the common knowledge in the art.

**EXAMPLE**

Hereinafter, the solutions of the present application are described in further detail with the help of the examples, but it will be understood by the person skilled in the art that the protection scope of the present application is not limited hereto.
Unless otherwise specified, various reagents, materials and equipment used in the following Examples are commercially available. Unless otherwise specified, molecular biology techniques involved in the following Examples can be found in, for example, Sambrook et al., Molecular Cloning, A Laboratory Manual, Cold Springs Harbor Press (Cold Springs Harbor, NY 1989).

**Example 1:** Design, expression and purification of fusion protein constructs

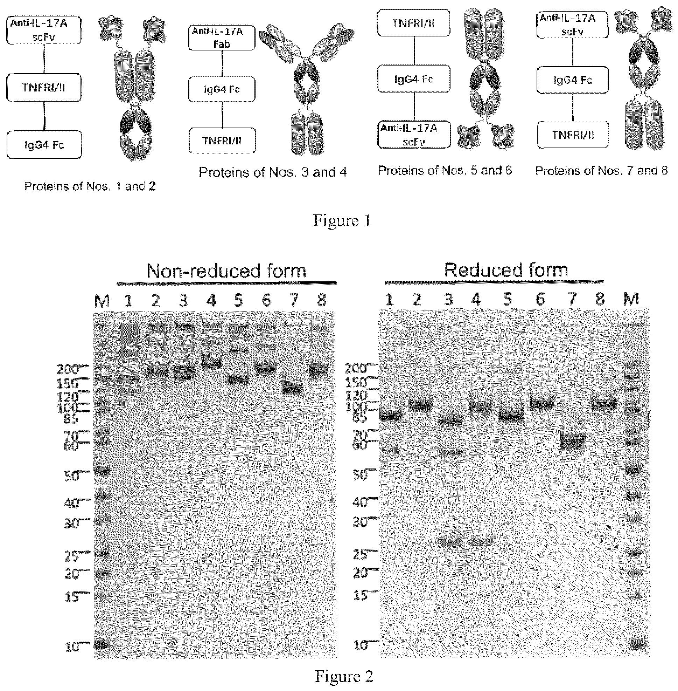

Based on the sequences of the extracellular truncated regions of sTNFRI or sTNFRII disclosed in the NCBI database, as well as the anti-IL-17A scFv or IL-17A Fab, eight constructs were obtained by fusion through the Fc fragment (S228P) of IgG4. For the convenience of description, the subsequent proteins are numbered sequentially, abbreviated as proteins of Nos. 1-8. The odd-numbered proteins (i.e., Nos. 1, 3, 5 and 7) are the constructs containing sTNFRI, while the even-numbered proteins (i.e., Nos. 2, 4, 6 and 8) are the constructs containing sTNFRII, which correspond to the four structures in Figure 1. The construct of each fusion protein is shown in Figure 1, wherein the sequences used are as follows:

The amino acid sequence of sTNFRI is set forth in SEQ ID NO: 1;

The amino acid sequence of sTNFRII:

LPAQVAFTPYAPEPGSTCRLREYYDQTAQMCCSKCSPGQHAKVFCTKTSDTVCDSCEDST
YTQLWNWVPECLSCGSRCSSDQVETQACTREQNRICTCRPGWYCALSKQEGCRLCAPLRK
CRPGFGVARPGTETSDVVCKPCAPGTFSNTTSSTDICRPHQICNVVAIPGNASMDAVCTSTS
PTRSMAPGAVHLPQPVSTRSQHTQPTPEPSTAPSTSFLLPMGPSPPAEGSTGD (SEQ ID NO: 13);

The amino acid sequence of anti-IL-17A scFv is set forth in SEQ ID NO: 3;

The amino acid sequence of anti-IL-17A Fab:

Heavy chain:

QVQLVQSGAEVKKPGSSVKVSCKASGYSFTDYHIHWVRQAPGQGLEWMGVINPMYGTTD
YNQRFKGRVTITADESTSTAYMELSSLRSEDTAVYYCARYDYFTGTGVYWGQGTLVTVSS
ASTKGPSVFPLAPCSRSTSESTAALGCLVKDYFPEPVTVSWNSGALTSGVHTFPAVLQSSGL
YSLSSVVTVPSSSLGTKTYTCNVDHKPSNTKVDKRV (SEQ ID NO: 14);

Light chain:

DIVMTQTPLSLSVTPGQPASISCRSSRSLVHSRGNTYLHWYLQKPGQSPQLLIYKVSNRFIG
VPDRFSGSGSGTDFTLKISRVEAEDVGVYYCSQSTHLPFTFGQGTKLEIKRTVAAPSVFIFPP
SDEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 15);

The amino acid sequence of the Fc fragment (S228P) of IgG4 is set forth in SEQ ID NO: 2.

The eight proteins were transiently expressed using the Expi293 system (purchased from Thermo Fisher) according to the manufacturer's instruction thereof, and the expression products were purified by Protein A affinity chromatography (resin: Praesto Jetted A50; equilibration: 50 mM Tris-HAc, 110 mM NaCl, pH 7.2; elution: 50 mM NaAc-HAc, pH 3.0). The eight proteins were analyzed by SDS-PAGE (reduced/non-reduced). The results are shown in Figure 2.

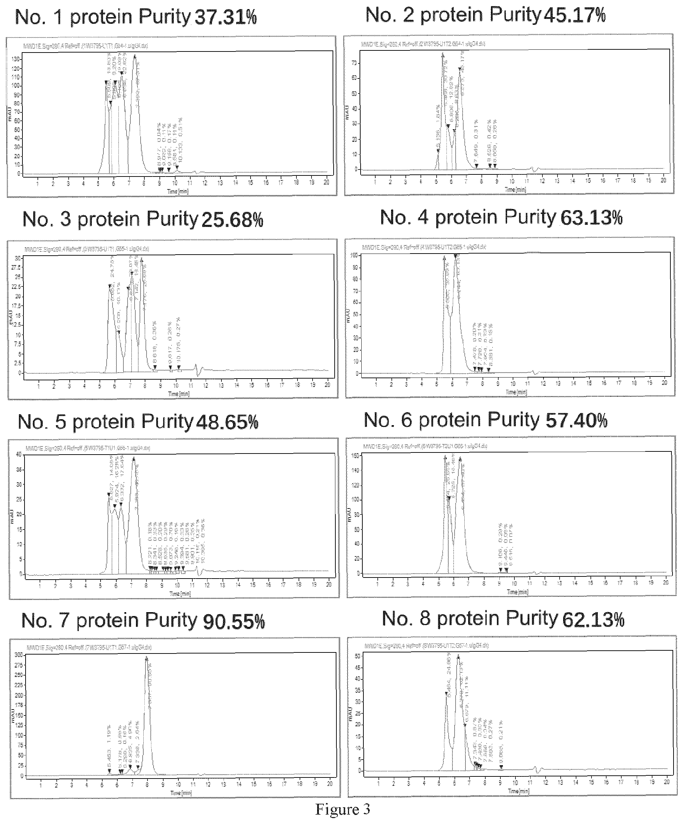

After protein A affinity chromatography was completed for all eight proteins, through SEC-HPLC (column: TSKgel® G3000SWXL; mobile phase A: 100 mM PB, 100 mM $Na_2SO_4$, pH 6.7±0.1; mobile phase B: 100% $H_2O$; flow rate: 0.7 mL/min; injection volume: 1.5 μL) analysis, it was found that except for No. 7 protein, which had a higher purity, there were different proportions of aggregates and degradation products in other proteins, as shown in Figure 3.

**Example 2:** Affinity assessment for eight proteins with different constructs

To further assess the eight proteins obtained in Example 1, except for No. 7 protein whose purity was sufficient for affinity analysis, the remaining proteins were further performed a second purification. Molecular-exclusion chromatography (resin: Sephadex G-25; buffer: PB) was utilized to obtain proteins with further improved purity. The sample amounts, concentrations, and purities of eight proteins after purification are shown in Table 1.

Table 1. Data of eight proteins after purification

| Protein Name | The sample amount (mg) | Concentration (mg/mL) | Purity | The sample amount (mg) |
|---|---|---|---|---|
| No. 1 protein | 1.55 | 1.55 | 88.34% | 1.55 |
| No. 2 protein | 1.28 | 1.28 | 76.14% | 1.28 |
| No. 3 protein | 0.35 | 0.43 | 49.18% | 0.35 |
| No. 4 protein | 0.58 | 0.52 | 71.83% | 0.58 |
| No. 5 protein | 0.65 | 0.65 | 95.39% | 0.65 |
| No. 6 protein | 3.39 | 1.13 | 95.05% | 3.39 |
| No. 7 protein | 7.75 | 1.99 | 90.55% | 7.75 |
| No. 8 protein | 0.88 | 0.68 | 74.03% | 0.88 |

The antigenic affinities of the above eight proteins with different constructs were analyzed by SPR technology (Biacore T200), and compared with the antigenic affinities of the positive control drugs Humira, Etanercept, and Cosentyx.

The above eight proteins and the control drugs Humira, Etanercept, and Cosentyx were bound onto the protein A sensor chip of Biacore T200 via Fc fragment, respectively. Then different concentrations (200 nM, 100 nM, 50 nM, 25 nM, 12.5 nM, 6.25 nM, 3.125 nM, 0 nM) of TNF or IL-17A were used as analytes. The affinities of the different proteins and control drugs for these two antigens were detected by multi-cycle method. The results showed that the affinities of seven proteins (proteins of Nos. 1, 2, 3, 4, 5, 6, and 8) for TNF-$\alpha$ were one order of magnitude higher than that of Humira (anti-TNF mAb), close to Etanercept (TNF receptor 2), which was in line with theoretical predictions, and No. 7 protein was close to Humira; and the affinities of the eight proteins for IL-17A were one order of magnitude higher than that of Cosentyx. The results of the antigen affinity assay are shown in Table 2.

Table 2. Affinities of the eight proteins and three positive control drugs for the response antigen

| Protein Name | Affinity (TNF$\alpha$) | | | Affinity (IL-17A) | | |
|---|---|---|---|---|---|---|
| | Ka (1/M·s) | Kd (1/s) | KD (M) | Ka (1/M·s) | Kd (1/s) | KD (M) |
| No. 1 protein | 1.872E+6 | 4.922E-5 | 2.629E-11 | 8.620E+6 | 2.808E-5 | 3.257E-12 |
| No. 2 protein | 6.270E+6 | 2.450E-4 | 3.908E-11 | 9.410E+6 | 3.011E-5 | 3.200E-12 |
| No. 3 protein | 3.233E+6 | 1.232E-4 | 3.809E-11 | 1.103E+7 | 9.568E-5 | 8.678E-12 |
| No. 4 protein | 5.124E+6 | 2.742E-4 | 5.351E-11 | 1.081E+7 | 7.745E-5 | 7.165E-12 |
| No. 5 protein | 2.191E+6 | 7.822E-5 | 3.571E-11 | 5.156E+6 | 3.282E-5 | 6.366E-12 |
| No. 6 protein | 6.573E+6 | 2.621E-4 | 3.987E-11 | 5.457E+6 | 4.809E-5 | 8.812E-12 |
| No. 7 protein | 2.183E+6 | 2.592E-4 | 1.187E-10 | 1.020E+7 | 6.393E-5 | 6.267E-12 |
| No. 8 protein | 7.284E+6 | 2.640E-4 | 3.624E-11 | 1.037E+7 | 8.072E-5 | 7.785E-12 |
| Humira | 9.41E+5 | 1.496E-4 | 1.599E-10 | | | |
| Etanercept | 1.058E+7 | 1.865E-4 | 1.763E-11 | | | |
| Cosentyx | | | | 7.775E+5 | 6.118E-5 | 7.869E-11 |

Since the fusion protein is a dual-target protein, it is necessary to consider whether the two targets have an effect on the binding of the antigen (TNF$\alpha$ or IL-17A). Therefore, a follow-up test was conducted using Biacore T200, that is, protein A chip was used to capture different proteins and positive control drugs, then one antigen was used to saturate the binding of a protein or a control drug to the antigen. Another antigen with different concentrations (200 nM, 100 nM, 50 nM, 25 nM, 12.5 nM, 6.25 nM, 3.125 nM, 0 nM) was used to detect the affinity of the protein or the positive control drug for said another antigen in this situation. Results are shown in Tables 3 and 4. The results showed that the affinities of the eight proteins obtained in Example 1 of the present application for the binding to another antigen is not affected when one antigen-binding domain is saturated.

Table 3. Affinities of eight proteins and control drugs for TNF$\alpha$ after saturation binding with IL-17A

| Protein Name | Affinity (TNF$\alpha$) | | | IL-7A MAX-Affinity (TNF$\alpha$) | | |
|---|---|---|---|---|---|---|
| | Ka (1/M·s) | Kd (1/s) | KD (M) | Ka (1/M·s) | Kd (1/s) | KD (M) |
| No. 1 protein | 1.872E+6 | 4.922E-5 | 2.629E-11 | 5.314E+6 | 1.107E-4 | 2.083E-11 |
| No. 2 protein | 6.270E+6 | 2.450E-4 | 3.908E-11 | 1.129E+7 | 5.488E-4 | 4.860E-11 |
| No. 3 protein | 3.233E+6 | 1.232E-4 | 3.809E-11 | 5.695E+6 | 1.035E-4 | 1.818E-11 |
| No. 4 protein | 5.124E+6 | 2.742E-4 | 5.351E-11 | 9.621E+6 | 5.919E-4 | 6.153E-11 |
| No. 5 protein | 2.191E+6 | 7.822E-5 | 3.571E-11 | 5.544E+6 | 9.486E-5 | 1.711E-11 |
| No. 6 protein | 6.573E+6 | 2.621E-4 | 3.987E-11 | 1.141E+6 | 4.573E-4 | 4.009E-11 |
| No. 7 protein | 2.183E+6 | 2.592E-4 | 1.187E-10 | 6.515E+6 | 5.053E-4 | 7.756E-11 |
| No. 8 protein | 7.284E+6 | 2.640E-4 | 3.624E-11 | 9.999E+6 | 2.945E-4 | 2.945E-11 |
| Humira | 9.41E+5 | 1.496E-4 | 1.599E-10 | | | |
| Etanercept | 1.058E+7 | 1.865E-4 | 1.763E-11 | | | |

Table 4. Affinities of eight proteins and control drugs for IL-17A after saturation binding with TNF$\alpha$

| Protein Name | Affinity (IL-7A) | | | TNF$\alpha$ MAX-Affinity (IL-17A) | | |
|---|---|---|---|---|---|---|
| | Ka (1/M·s) | Kd (1/s) | KD (M) | Ka (1/M·s) | Kd (1/s) | KD (M) |
| No. 1 protein | 8.620E+6 | 2.808E-5 | 3.257E-12 | 7.666E+6 | 3.278E-5 | 4.276E-12 |
| No. 2 protein | 9.410E+6 | 3.011E-5 | 3.200E-12 | 8.620E+6 | 2.999E-5 | 3.479E-12 |
| No. 3 protein | 1.103E+7 | 9.568E-5 | 8.678E-12 | 8.636E+6 | 5.091E-5 | 5.895E-12 |
| No. 4 protein | 1.081E+7 | 7.745E-5 | 7.165E-12 | 8.410E+6 | 6.205E-5 | 7.379E-12 |
| No. 5 protein | 5.156E+6 | 3.282E-5 | 6.366E-12 | 3.637E+6 | 2.603E-5 | 7.158E-12 |
| No. 6 protein | 5.457E+6 | 4.809E-5 | 8.812E-12 | 3.902E+6 | 3.393E-5 | 8.695E-12 |
| No. 7 protein | 1.020E+7 | 6.393E-5 | 6.267E-12 | 5.801E+6 | 4.760E-5 | 8.206E-12 |
| No. 8 protein | 1.037E+7 | 8.072E-5 | 7.785E-12 | 6.060E+6 | 4.736E-5 | 7.816E-12 |
| Cosentyx | 7.775E+5 | 6.118E-5 | 7.869E-11 | | | |

By combining with the results of expression and purification of the eight proteins assayed in Example 1 and the above affinity assessment results in Example 2, and according to the chip response values of different proteins and control drugs binding to antigen molecules, whether each protein or control drug can guarantee the ratio of binding to TNF and IL-17A was calculated. Theoretically, the ratio of binding to TNF and IL-17A is 1:2. According to the following equation, the mole ratio (Ratio) of binding to antigens TNF$\alpha$ and IL-17A by the eight fusion proteins prepared in Example 1 and the positive control drugs with the same mole number was calculated by chip response values:

$$Ratio = \frac{Analyte\ (Ru)}{Analyte\ MW(KDa)} \Big/ \frac{Ligand\ (Ru)}{Ligand\ MW(KDa)}$$

Wherein Analyte represents TNF$\alpha$ or IL-17A; Ligand represents the proteins or positive control drugs anchored on the chip: the eight proteins and the positive control antibodies; Analyte MW represents the relative molecular mass of the analyte; Ligand MW represents the relative molecular mass of the proteins or positive control drugs anchored on the chip.

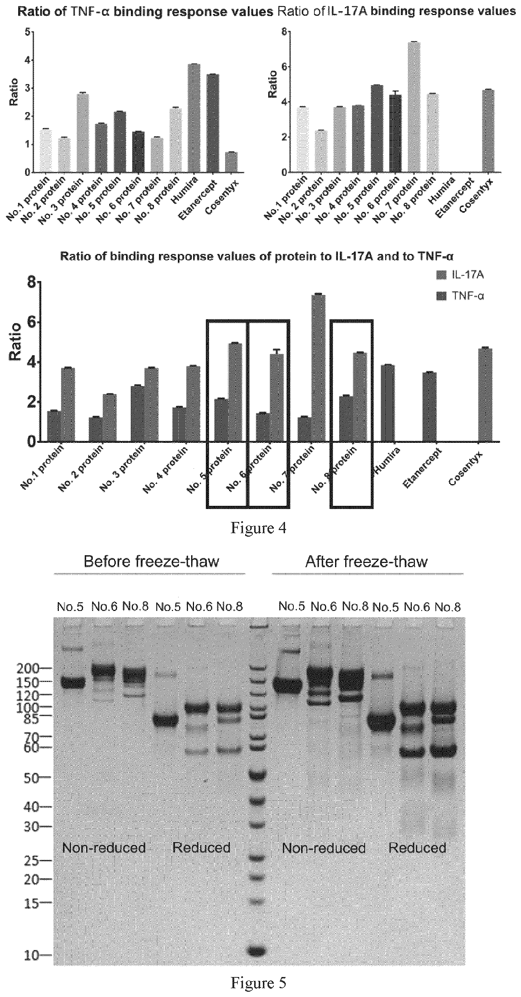

The results of the response values of the above eight proteins and the control drugs binding to antigen are shown in Figure 4. Based on the above sample amounts, purities, and affinities of proteins in Example 1 and Example 2, proteins of Nos. 5, 6, and 8 were selected for the next mass spectrometric detection.

**Example 3:** Mass spectrometric detection analysis of proteins of Nos. 5, 6, and 8

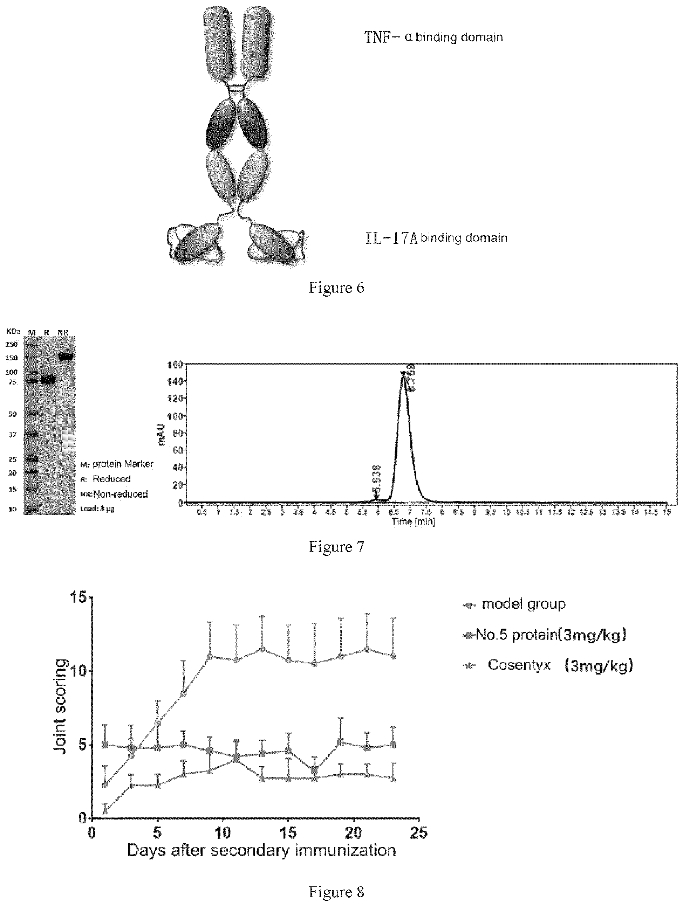

After freezing at -80°C and thawing, it was found by SDS-PAGE analysis that a large number of degradation bands appeared in proteins of Nos. 6 and 8, as shown in Figure 5. According to the mass spectrometry detection, only No. 5 protein has a complete main peak; proteins of Nos. 6 and 8 have unknown peaks, and the actual molecular masses thereof differ greatly from the theoretical values, as shown in Table 5. Therefore, No. 5 protein was selected as the candidate molecule for further experiments. The schematic diagram of the construct of No. 5 protein is shown in Figure 6.

Table 5. Results of mass spectrometric detection of proteins of Nos. 5, 6 and 8

| Sample Name | Type | Theoretical molecular weight (Da) | Measured molecular weight (Da) | Difference (ppm) | Relative content (%) |
|---|---|---|---|---|---|
| No. 5 protein | Single strand: complete denaturation, deglycosylation | 71919.8 | 71916.5 | -45.9 | NA |
| | Single strand: complete denaturation, deglycosylation, breakage (5-657) | 71350.1 | 71348.4 | -23.8 | 3%* |
| No. 6 protein | unknown peak 1 | 78792.5 | 53879.0 | NA | Main peak |
| | unknown peak 2 | NA | 48178.4 | NA | Low ratio, <1%# |
| | unknown peak 3 | NA | 20087.0 | NA | Low ratio, <1%# |
| No. 8 protein | unknown peak 4 | 78775.5 | 54326.5 | NA | Main peak (3:1) |
| | unknown peak 5 | NA | 73931.0 | NA | |
| | unknown peak 6 | NA | 27235.5 | NA | Low ratio, <1%# |
| | Single strand: complete denaturation, deglycosylation, breakage (178-352) | 19621.3 | 19620.4 | -45.9 | Low ratio, <1%# |

**Example 4:** Transient transfection expression of No. 5 protein in CHO Cells

Since the previous transient expression of the protein was performed using the Expi293 system, the final product of protein expression may be different from the product expressed by CHO cells. According to the manufacturer's instructions, No. 5 protein was expressed and purified by ExpiCHO system (purchased from Thermo Fisher), and the obtained protein was used for pharmacodynamic study and druggability analysis. Table 6 shows the relevant information of the protein obtained finally. Figure 7 shows the SEC-HPLC results and SDS-PAGE results of the protein obtained finally.

Table 6. Relevant information of the protein obtained finally

| | |
|---|---|
| Formulation | PBS, pH 7.4 |
| Purity measured by reducing SDS-PAGE | >90% |
| Purity measured by SEC-HPLC | 97.9% |
| Endotoxin (LAL) | <0.5 EU/mg |
| Yield of protein | 248.6 mg |
| Concentration of protein | 1.10 mg/mL |
| Volume | 226.0 mL |
| Numbers of vial | 68 |
| Storage and transportation | -80°C |

**Example 5:** Pharmacodynamic assessment of No. 5 protein

The protein obtained in Example 4 was used for pharmacodynamic study of the fusion protein. First, the affinities of No. 5 fusion protein for murine TNF-$\alpha$ (mTNF$\alpha$) and murine IL-17A (mIL-17A) was detected. The results showed that No. 5 fusion protein bound to mTNF$\alpha$ with an affinity constant of 1.630E-10 M, but did not bind to mIL-17A. Therefore, it is necessary to use hIL-17A transgenic mice for pharmacodynamic tests.

The hIL-17A transgenic DBA/1 mice were used, free access to water and food. After adaptive feeding for 3-5 days, the mice were randomly divided into control group and model group according to the body weight. Model group mice were administered with bovine type II collagen (Chondrex company) emulsion formulated with complete Freund's adjuvant by subcutaneous injection at the tail root. Bovine type II collagen emulsion formulated with incomplete Freund's adjuvant was used for secondary immunization 21 days after the primary immunization. 42-56 days after the primary immunization, the success rate of mice modeling can reach 100%; and 30 days after the primary immunization, the mice were divided into model group (administered with PB buffer) (n=4), blank group (n=3), No. 5 fusion protein group (n=5), positive control drug Cosentyx group (n=4). To validate potency, when grouping, the most severe, high-scoring modeled mice were divided into the fusion protein group to observe the inhibitory effect of the fusion protein on the modeled mice in the group.

Treatment was began on the grouping day and administered once every 48h, with No. 5 fusion protein at a concentration of 1.1 mg/mL, Cosentyx at a concentration of 1.1 mg/mL, 80 $\mu$L/mouse. The way of administration was intraperitoneal injection, and the dose of administration was 3 mg/kg. The degree of joint swelling of mice was scored before each administration, and the scoring criteria are shown in Table 7 below.

Table 7 Joint scoring criteria

| Score | Criteria |
| --- | --- |
| 0 | Normal |
| 1 | Not obvious, tangible swelling |
| 2 | Slight swelling is visible to the naked eye |
| 3 | Obvious swelling is visible to the naked eye |
| 4 | Rigid joints |

Photos were taken to record and the change curve was plotted. The treatment period was 28 days, with a total of 14 doses administered. The results of joint scoring of mice are shown in Figure 8.

In the administration group of No. 5 protein or Cosentyx, No. 5 fusion protein has a significant inhibitory effect on rheumatoid arthritis in mice, and the inhibitory effect is better than that of positive control drug Cosentyx. The inhibition rate of rheumatoid arthritis was calculated according to the following equation:

$$T = \text{Final score of the administration group - Initial score of the administration group}$$

$$C = \text{Final score of the model group - Initial score of the model group}$$

$$\text{Inhibition rate of rheumatoid arthritis} = (1-T/C) *100\%$$

The inhibition rate of rheumatoid arthritis in each administration group was obtained via calculation, which was as follows:

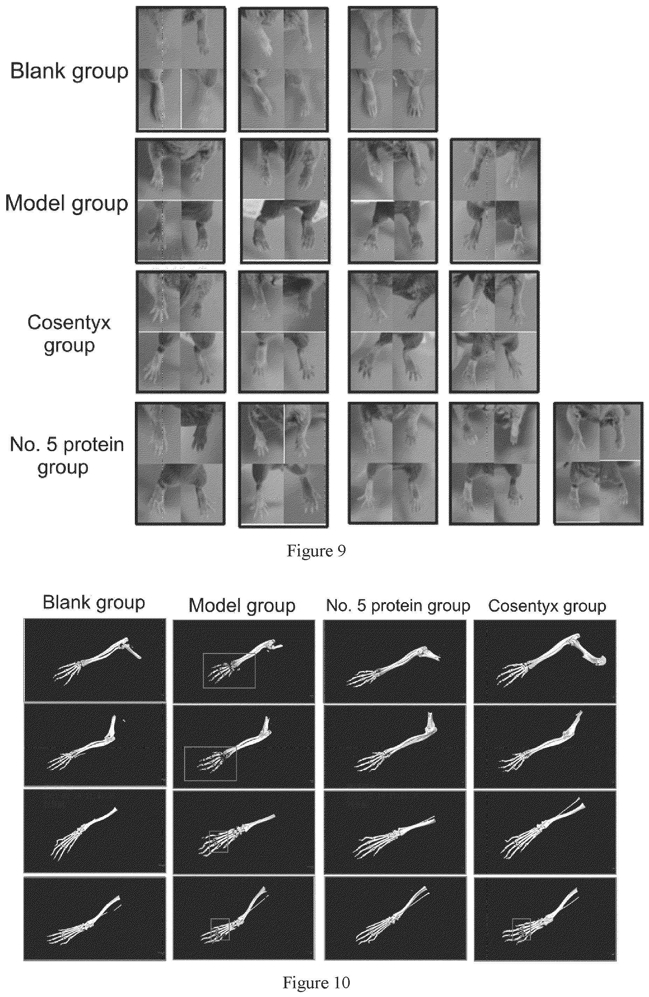

The inhibition rate of No. 5 fusion protein: 74%; the inhibition rate of Cosentyx: 40% (the photos of the paws of the mice in each group are shown in Figure 9).

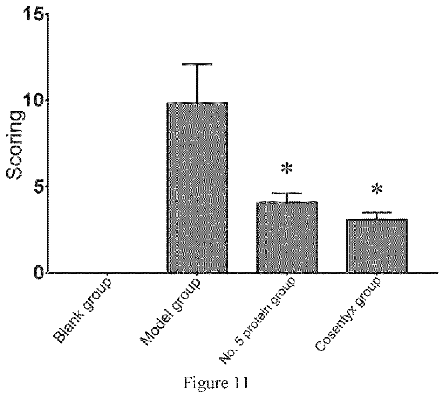

Samples were collected from the paws of mice in each group, and MicroCT analysis was performed, and the degree of bone injury of the paws of mice in each group was scored. Three laboratory technicians scored them back-to-back according to the criteria in Table 7, and took the average value. The MicroCT results of paws and scores of mice in each group are shown in FIG. 10 and FIG. 11. The results showed that No. 5 fusion protein can significantly alleviate osteoarticular injury, and the effect of Cosentyx was also good (partly because the scores of rheumatoid arthritis of mice in this administration group in the early stage were lower than those in the No. 5 protein administration group).

**Example 6:** Analyses of physicochemical properties of No. 5 fusion protein

(1) Differential Scanning Fluorimetry (DSF)

Differential scanning fluorimetry is a method to evaluate the thermal stability of proteins by slowly heating samples on a fluorescence quantitative PCR instrument and detecting the amount of fluorescent dye bound to proteins with structural changes during the heating process. Differential scanning fluorimetry was used to assay No. 5 protein, and No. 5 protein at a concentration of 1 mg/mL was detected in two wells; the average value of Tm was 68.6°C, and the results of assay are shown in Table 8, indicating that the sample has good thermal stability; but fluorescence was observed at the beginning of the detection, indicating the exposure of patches of hydrophobic groups on the protein surface or the presence of protein misfolding.

Table 8. Results of differential scanning fluorimetry of No. 5 protein

| Protein | Well | pI | Buffer | Concentration (mg/mL) | Tm1 (°C) | Tm2 (°C) | Comment |
|---|---|---|---|---|---|---|---|
| No. 5 protein | 1 | 8.1 | PBS | 1.1 | 68.5 | - | Initial fluorescence |
| | 2 | | | | 68.8 | - | |

(2) Detection of FcRn binding ability

The affinity of No. 5 protein for FcRn under the condition of pH 7.4 was detected by using protein A chip through SPR technology. After analysis, its affinity constant was 2.52E-06 nM, and this affinity detection result fell within the normal range of the affinity of IgG4 subtype antibody for FcRn, indicating that No. 5 protein can extend the half-life of the fusion protein in the body through Fc.

(3) Detecting the plasma stability of No. 5 protein by the method of ELISA

No. 5 protein was incubated in human plasma at 37°C for 0 days (i.e., one day before incubation), 1 day, 4 days, 7 days and 14 days. Then the binding ability of No. 5 protein to hTNF-$\alpha$ and hIL-17A was detected by ELISA (No. 5 protein was used as the primary antibody and HRP-labeled anti-human Fc was used as the secondary antibody). The results showed that the EC50 values after incubation for 1 day, 4 days, 7 days and 14 days were almost unchanged from the EC50 values on day 0, indicating that No. 5 protein was stable in human plasma.

(4) Dynamic Light Scattering (DLS) Test

Dynamic light scattering test was used to detect the distribution of proteins of different sizes in the solution of No. 5 protein with a concentration of 1.10mg/mL in PBS buffer (pH 7.4). The solution of No. 5 protein in PBS buffer was tested twice. Through the test, PD% (relative dispersion) of the solution was >15%, indicating that oligomers of No. 5 protein existed in the solution. The results are shown in Table 9.

Table 9. DLS results of No. 5 protein

| Sample | Run | DLS temperature (°C) | Radius (nm) | PD % | Mean radius (nm) | Average PD% |
|---|---|---|---|---|---|---|
| No. 5 protein | 1 | 25 | 11.6 | 16.9 | 11.65 | 16.7 |
| | 2 | 25 | 11.7 | 16.5 | | |

(5) Detection of freeze-thaw stability of No. 5 protein

The sample of No. 5 protein on day 0 (i.e., one day before incubation) was freeze-thawed once at <-60°C after purification. The other samples were incubated at 40°C (incubated 14 days in total, detected on day 1, 4, 7 and 14) or performed for three rounds of freeze-thaw (<-60°C). Through A280 detection, the concentrations of the samples are close, and the appearance is colorless with particle-free. By detecting with SEC-HPLC method (chromatographic column: TSKgel® G3000SWXL; mobile phase A: 100 mM PB, 100 mM $Na_2SO_4$, pH 6.7$\pm$0.1; mobile phase B: 100% $H_2O$; flow rate: 0.7 mL/min; injection volume: 1.5 $\mu$L), the presence of high molecular weight components (aggregates) was found in all samples. It is worth noting that after incubation at 40°C, the high molecular weight components reduced and the monomer components increased. The detection results of freeze-thaw stability are shown in Table 10. The results showed that No. 5 protein had certain freeze-thaw stability.

Table 10. Results of freeze-thaw stability of No. 5 protein

| No. | Protein Name | Treatment | Concentration (mg/mL) | HMW % | monomer % | LMW % | appearance |
|---|---|---|---|---|---|---|---|
| 1 | No. 5 protein-T0 | freeze-thaw, day 0 | 1.07 | 57.02 | 42.46 | 0.52 | CL PF |
| 2 | No. 5 protein-3C | three rounds of freeze-thaw | 0.76 | 57.05 | 42.89 | 0.06 | CL PF |
| 3 | No. 5 protein-T1 | 40°C, day 1 | 1.09 | 48.72 | 50.96 | 0.32 | CL PF |
| 4 | No. 5 protein-T4 | 40°C, day 4 | 1.07 | 31.27 | 67.22 | 1.51 | CL PF |
| 5 | No. 5 protein-T7 | 40°C, day 7 | 1.07 | 13.90 | 80.56 | 5.54 | CL PF |
| 6 | No. 5 protein-T14 | 40°C, day 14 | 1.09 | 22.92 | 73.82 | 3.26 | CL PF |
| Note: colorless (CL), particle free (PF); HMW: High molecular weight; LMW: Low molecular weight | | | | | | | |

Example 7: Formulation formula screening

Since it was found in the previous experiments that candidate molecules may produce aggregates after freeze-thaw, in order to further improve the freeze-thaw stability of the fusion protein in the present application and further reduce the production of aggregates, thereby further improving its druggability, different formulas and pH values suitable for No. 5 protein were studied in this Example.

The pH range of the buffer system was designed (4.5~7.5) according to the theoretical isoelectric point (8.07) of No. 5 fusion protein. Meanwhile, the effects of different kinds of buffer systems (acetate, histidine, citrate and phosphate) on the stability of the protein were investigated. The types and amounts of proteins, excipients and surfactants added in each candidate formula were the same. By testing the appearance and visible foreign matters as well as DLS and DSC, comparing the differences in particle, colloidal stability and thermal stability of candidate molecules in different candidate formulas, 3-6 optimal formulas were screened out for freeze-thaw investigation. Specific protocol is shown in Table 11.

Table 11. Formulas of formulation and detection indexes of No. 5 protein

| Sample No. | pH/Buffer system | Content of protein | Excipient | Surfactant | Detection indexes |
|---|---|---|---|---|---|
| B-1 | 10 mM acetic acid/sodium acetate, pH 4.5±0.2 | 20 mg/mL | 87 mg/mL trehalose | 0.02% (w/v) polysorbate 80 | X |
| B-2 | 10 mM acetic acid/sodium acetate, pH 5.0±0.2 | | | | |
| B-3 | 10 mM acetic acid/sodium acetate, pH 5.5±0.2 | | | | |
| B-4 | 10 mM citric acid/sodium citrate, pH 5.0±0.2 | | | | |
| B-5 | 10 mM citric acid/sodium citrate, pH 5.5±0.2 | | | | |
| B-6 | 10 mM citric acid/sodium citrate, pH 6.0±0.2 | | | | |
| B-7 | 10 mM histidine/histidine hydrochloride, pH 5.5±0.2 | | | | |
| B-8 | 10 mM histidine/histidine hydrochloride, pH 6.0±0.2 | | | | |

(continued)

| Sample No. | pH/Buffer system | Content of protein | Excipient | Surfactant | Detection indexes |
|---|---|---|---|---|---|
| B-9 | 10 mM histidine/histidine hydrochloride, pH 6.5±0.2 | | | | |
| B-10 | 10 mM disodium hydrogen phos-phate/sodium dihydrogen phos-phate, pH 6.5±0.2 | | | | |
| B-11 | 10 mM disodium hydrogen phos-phate/sodium dihydrogen phos-phate, pH 7.0±0.2 | | | | |
| B-12 | 10 mM disodium hydrogen phos-phate/sodium dihydrogen phos-phate, pH 7.5±0.2 | | | | |
| X: appearance, visible foreign matters, DSC, DLS (Tagg & kD). | | | | | |

After detection, the results of the fast screening study of the above formulas showed that the kD values of B-1 to B-3 and B-7 to B-9 were >0 in the samples of each candidate formula, indicating that the sample molecules of each formula were mainly repulsive to each other, wherein B-1 showed the best performance; B-1 to B-3 and B-7 to B-10 had higher Tagg (aggregation initial temperature) values and had relatively higher thermal stability; B-2, B-3, B-5 and B-6 had higher Tonset (denaturation initial temperature) values and had relatively higher thermal stability; and the appearance and particle situation of samples of the formulas B-1 and B-8 were relatively better.

In summary, B-1 to B-3 and B-7 to B-8 were more conducive to maintaining the stability of No. 5 fusion protein of the present application. These 5 formulas were finally and preferably selected as candidate formulation systems. In addition, the experimental group of "buffer system + sucrose" of B-1 formula with the best performance (10 mM acetic acid/sodium acetate buffer (pH 4.5), 80 mg/mL sucrose, 0.02% polysorbate 80) was added. The above candidate formulation systems were investigated by freeze-thaw. The experimental protocol is shown in Table 12.

Table 12. Candidate formulation systems

| Sample No. | Content of protein | pH/ Buffer system | Excipient | Surfactant |
|---|---|---|---|---|
| E-1 | 20 mg/mL | 10 mM acetic acid/sodium acetate, pH 4.5±0.2 | 87 mg/mL trehalose | 0.02% (w/v) polysorbate 80 |
| E-2 | | 10 mM acetic acid/sodium acetate, pH 5.0±0.2 | | |
| E-3 | | 10 mM acetic acid/sodium acetate, pH 5.5±0.2 | | |
| E-4 | | 10 mM histidine/histidine hydrochloride, pH 5.5±0.2 | | |
| E-5 | | 10 mM histidine/histidine hydrochloride, pH 6.0±0.2 | | |
| E-6 | | 10 mM acetic acid/sodium acetate, pH 4.5±0.2 | 80 mg/mL sucrose | |

The investigation results of freeze-thaw showed that compared to other candidate formulation systems, the formula E-1 (10 mM acetic acid/sodium acetate buffer, 87 mg/mL trehalose and 0.02% (w/v) polysorbate 80, pH 4.5) was more conducive to maintaining the stability of the fusion protein of the present application, and had the best overall performance.

Those skilled in the art will recognize that the scope of this application is not limited to various specific embodiments and examples described above, but various modifications, substitutions, or re-combinations can be made without departing from the spirit of this application, all of which fall within the protection scope of the present application.

**Claims**

1. A bispecific fusion protein, comprising, in the order from N-terminal to C-terminal:

a soluble TNF receptor or a portion thereof;
a human IgG Fc fragment; and
a functional domain competitively binding to IL-17A or against IL-17A.

2. The bispecific fusion protein of claim 1, wherein the soluble TNF receptor or the portion thereof is soluble TNF receptor 1 or a portion thereof, or soluble TNF receptor 2 or a portion thereof; more

preferably, the soluble TNF receptor or the portion thereof is soluble TNF receptor 1 or a portion thereof; more preferably, the soluble TNF receptor or the portion thereof is an extracellular fragment of soluble TNF receptor 1; preferably, the extracellular fragment of soluble TNF receptor 1 comprises an amino acid sequence set forth in SEQ ID NO: 1 or an amino acid sequence having at least 95% identity thereto, or a conservatively modified variant thereof.

3. The bispecific fusion protein of claim 1 or 2, wherein the human IgG Fc fragment is a human IgG1 Fc or a human IgG4 Fc fragment; more preferably, the human IgG Fc fragment is a Hinge-CH2-CH3 fragment of IgG4; more preferably, the human IgG Fc fragment is a human IgG4 Fc fragment with S228P mutation.

4. The bispecific fusion protein of any one of claims 1-3, wherein the functional domain competitively binding to IL-17A or against IL-17A is selected from: a receptor of IL-17A, a variant thereof or a portion thereof, or an anti-IL-17A antibody or an antigen-binding fragment thereof;

preferably, the antigen-binding fragment of the anti-IL-17A antibody comprises a Fab fragment, a Fab' fragment, a F(ab')2 fragment, an Fv fragment, a single-chain fragment variable, a single-domain antibody, an isolated CDR region or an Fd fragment of the anti-IL-17A antibody; preferably, the functional domain competitively binding to IL-17A or against IL-17A is an anti-IL-17A single-chain fragment variable; preferably, the anti-IL-17A single-chain fragment variable comprises: a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10; preferably, the anti-IL-17A single-chain fragment variable comprises: a heavy chain variable region comprising an amino acid sequence set forth in SEQ ID NO: 11 or an amino acid sequence having at least 80% identity thereto, and a light chain variable region comprising an amino acid sequence set forth in SEQ ID NO: 12 or an amino acid sequence having at least 80% identity thereto; preferably, the anti-IL-17A single-chain fragment variable comprises an amino acid sequence set forth in SEQ ID NO: 3 or an amino acid sequence having at least 80% identity thereto.

5. The bispecific fusion protein of any one of claims 1-4, wherein the bispecific fusion protein comprises, in the order from N-terminal to C-terminal: (1) soluble TNF receptor 1 or 2, or an extracellular fragment thereof, (2) a human IgG4 Fc fragment, and (3) an anti-IL-17A single-chain fragment variable;

preferably, the bispecific fusion protein comprises, in the order from N-terminal to C-terminal: (1) soluble TNF receptor 1 or an extracellular fragment thereof, (2) a human IgG4 Fc fragment, and (3) an anti-IL-17A single-chain fragment variable; preferably, the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10; preferably, the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable comprising: a heavy chain variable region comprising an amino acid sequence set forth in SEQ ID NO: 11, and a light chain variable region comprising an amino acid sequence set forth in SEQ ID NO: 12; preferably, the bispecific fusion protein comprises, in the order from N-terminal to C-terminal: (1) an extracellular fragment of soluble TNF receptor 1 set forth in SEQ ID NO: 1, (2) a human IgG4 Fc fragment set forth in SEQ ID NO: 2, and (3) an anti-IL-17A single-chain fragment variable set forth in SEQ ID NO: 3.

6. The bispecific fusion protein of any one of claims 1-5, wherein the human IgG Fc fragment is linked to the functional domain competitively binding to IL-17A or against IL-17A via a linker peptide;

preferably, the linker peptide is a GS flexible linker peptide.

7. The bispecific fusion protein of any one of claims 1-6, wherein the bispecific fusion protein comprises, in the order from N-terminal to C-terminal: soluble TNF receptor 1 or an extracellular fragment thereof, a human IgG4 Fc fragment, a GS flexible linker peptide and an anti-IL-17A single-chain fragment variable;

preferably, the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a HCDR1 set forth in SEQ ID NO: 5, a HCDR2 set forth in SEQ ID NO: 6 and a HCDR3 set forth in SEQ ID NO: 7, as well as a LCDR1 set forth in SEQ ID NO: 8, a LCDR2 set forth in SEQ ID NO: 9 and a LCDR3 set forth in SEQ ID NO: 10;

preferably, the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4, and an anti-IL-17A single-chain fragment variable comprising a heavy chain comprising an amino acid sequence set forth in SEQ ID NO: 11 and a light chain variable region comprising an amino acid sequence set forth in SEQ ID NO: 12;

preferably, the bispecific fusion protein comprises, in the order from N-terminal to C-terminal: an extracellular fragment of soluble TNF receptor 1 set forth in SEQ ID NO: 1, a human IgG4 Fc fragment set forth in SEQ ID NO: 2, a GS flexible linker peptide set forth in SEQ ID NO: 4 and an anti-IL-17A single-chain fragment variable set forth in SEQ ID NO: 3;

preferably, the fusion protein has an affinity constant $K_D$ of $9 \times 10^{-12}$ M or less for IL-17A, and an affinity constant $K_D$ of $4 \times 10^{-11}$ M or less for TNF$\alpha$.

8. A polynucleotide, encoding the bispecific fusion protein of any one of claims 1-7.

9. A recombinant vector, comprising the polynucleotide of claim 8.

10. A host cell, comprising the polynucleotide of claim 8 or the recombinant vector of claim 9.

11. A method of preparing the bispecific fusion protein of any one of claims 1-7, comprising:

a) preparing a polynucleotide encoding the bispecific fusion protein;

b) constructing a recombinant vector by using the polynucleotide and an expression vector;

c) transferring the recombinant vector into host cells and culturing transformed cells to obtain cell culture; and

d) purifying and isolating the cell culture to obtain the bispecific fusion protein.

12. A formulation comprising the bispecific fusion protein of any one of claims 1-7, wherein the formulation further comprises a buffer system, a pharmaceutically acceptable excipient and a surfactant, in which the buffer system is selected from a solution of acetic acid and sodium acetate, a solution of citric acid and sodium citrate, or a solution of histidine and histidine hydrochloride;

preferably, the formulation has a pH value of 3.0-7.5;

preferably, the formulation comprises: 5 mg/mL-100 mg/mL of the bispecific fusion protein, 5 mM-50 mM of the buffer system, 50 mg/mL-100 mg/mL of the pharmaceutically acceptable excipient and 0.01%-0.05% (w/v) of the surfactant;

preferably, the buffer system is selected from a 5 mM-20 mM solution of acetic acid and sodium acetate, a 5 mM-20 mM solution of citric acid and sodium citrate, or a 5 mM-20 mM solution of histidine and histidine hydrochloride;

preferably, the pharmaceutically acceptable excipient is at least one of sucrose or trehalose;

preferably, the surfactant is a non-ionic surfactant.

13. A pharmaceutical composition, comprising the bispecific fusion protein of any one of claims 1-7.

14. The bispecific fusion protein of any one of claims 1-7, the formulation of claim 12 or the pharmaceutical composition of claim 13 for use in treating an inflammation-related disease;

preferably, the inflammation-related disease is an autoimmune disease or a cytokine release syndrome;

more preferably, the inflammation-related disease is rheumatoid arthritis, psoriasis, psoriatic arthritis, ankylosing spondylitis, ulcerative colitis, or a cytokine release syndrome caused by viral infections and immunomodulatory drugs.

15. The bispecific fusion protein of any one of claims 1-7, the formulation of claim 12 or the pharmaceutical composition of claim 13 for use as a reagent for binding and inhibiting TNF-$\alpha$ and IL-17A.

Figure 1

Figure 2

No. 1 protein Purity 37.31%

No. 2 protein Purity 45.17%

No. 3 protein Purity 25.68%

No. 4 protein Purity 63.13%

No. 5 protein Purity 48.65%

No. 6 protein Purity 57.40%

No. 7 protein Purity 90.55%

No. 8 protein Purity 62.13%

Figure 3

Figure 4

Figure 5

TNF−α binding domain

IL−17A binding domain

Figure 6

M: protein Marker
R: Reduced
NR: Non-reduced
Load: 3 μg

Figure 7

- model group
- No.5 protein(3mg/kg)
- Cosentyx (3mg/kg)

Figure 8

Blank group

Model group

Cosentyx group

No. 5 protein group

Figure 9

Blank group    Model group    No. 5 protein group    Cosentyx group

Figure 10

Figure 11

## INTERNATIONAL SEARCH REPORT

| International application No. |
| --- |
| **PCT/CN2023/137845** |

| A. CLASSIFICATION OF SUBJECT MATTER |
| --- |
| C07K 19/00(2006.01)i; C12N 15/62(2006.01)i; A61K 39/395(2006.01)i; A61K 38/17(2006.01)i; A61P 29/00(2006.01)i; A61P 37/02(2006.01)i; A61P 19/02(2006.01)i |

According to International Patent Classification (IPC) or to both national classification and IPC

| B. FIELDS SEARCHED |
| --- |

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CNKI, PubMed, GenBank, 中国专利生物序列数据库, China Patents Biological Sequence Database: 双特异性, bi-specific, 融合蛋白, fusion protein, 肿瘤坏死因子受体, TNFR, IL-17A受体, IL-17AR, IL-17A抗体, SEQ ID NOs: 1-12

| C. DOCUMENTS CONSIDERED TO BE RELEVANT |
| --- |

| Category* | Citation of document, with indication, where appropriate, of the relevant passages | Relevant to claim No. |
| --- | --- | --- |
| X | CN 106892982 A (NORTHEAST AGRICULTURAL UNIVERSITY) 27 June 2017 (2017-06-27)<br>claims 1-10, and description, paragraphs 0009-0052 | 1-15 |
| X | CN 104311670 A (HARBIN BOAO BIOMEDICAL TECHNOLOGY DEVELOPMENT CO., LTD.) 28 January 2015 (2015-01-28)<br>claims 1-10, and description, paragraphs 0007-0014 | 1-15 |
| X | CN 107857818 A (SHANGHAI KEXIN BIOTECH CO., LTD.) 30 March 2018 (2018-03-30)<br>claims 1-9, and description, paragraphs 0005-0042 | 1-15 |
| X | CN 109796534 A (BEIJING BEYOND BIOTECHNOLOGY CO., LTD.) 24 May 2019 (2019-05-24)<br>claims 1-15 | 1-15 |
| A | CN 113474360 A (ELI LILLY AND CO.) 01 October 2021 (2021-10-01)<br>claims 1-17 | 1-15 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **14 March 2024** | **20 March 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/137845** |

### C. DOCUMENTS CONSIDERED TO BE RELEVANT

| Category* | Citation of document, with indication, where appropriate, of the relevant passages | Relevant to claim No. |
|---|---|---|
| A | CN 109679920 A (BEIJING BAYLX BIOTECH CO., LTD.) 26 April 2019 (2019-04-26) claims 1-18 | 1-15 |
| A | CN 111032691 A (ELI LILLY AND CO.) 17 April 2020 (2020-04-17) claims 1-55 | 1-15 |
| A | CN 113631574 A (NUMAB THERAPEUTICS AG) 09 November 2021 (2021-11-09) claims 1-15 | 1-15 |
| A | ELICABE, R. J. et al. "Lack of TNFR p55 Results in Heightened Expression of IFN-γ and IL-17 during the Development of Reactive Arthritis" *J Immunol.*, Vol. 185, No. 7, 01 October 2010 (2010-10-01), pages 4485-4495 | 1-15 |
| A | TERABE, F. et al. "Comparative Analysis of the Effects of Anti-IL-6 Receptor mAb and Anti-TNF mAb Treatment on CD4þ T-cell Responses in Murine Colitis" *Inflamm. Bowel Dis.*, Vol. 17, No. 2, 02 July 2010 (2010-07-02), pages 491-502 | 1-15 |

Form PCT/ISA/210 (second sheet) (July 2022)

## INTERNATIONAL SEARCH REPORT

| International application No. |
| --- |
| **PCT/CN2023/137845** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

    ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2023/137845**

| Patent document cited in search report | | | Publication date (day/month/year) | Patent family member(s) | | | Publication date (day/month/year) |
|---|---|---|---|---|---|---|---|
| CN | 106892982 | A | 27 June 2017 | None | | | |
| CN | 104311670 | A | 28 January 2015 | None | | | |
| CN | 107857818 | A | 30 March 2018 | US | 2020181236 | A1 | 11 June 2020 |
| | | | | WO | 2019029129 | A1 | 14 February 2019 |
| CN | 109796534 | A | 24 May 2019 | WO | 2019096026 | A1 | 23 May 2019 |
| | | | | US | 2021261654 | A1 | 26 August 2021 |
| | | | | US | 11667704 | B2 | 06 June 2023 |
| CN | 113474360 | A | 01 October 2021 | CO | 2021010697 | A2 | 29 October 2021 |
| | | | | MX | 2021009851 | A | 10 September 2021 |
| | | | | US | 2023183334 | A1 | 15 June 2023 |
| | | | | SG | 11202108627 | SA | 29 September 2021 |
| | | | | CR | 20210435 | A | 20 September 2021 |
| | | | | WO | 2020172002 | A1 | 27 August 2020 |
| | | | | PE | 20212185 | A1 | 11 November 2021 |
| | | | | EP | 3927729 | A1 | 29 December 2021 |
| | | | | EP | 3927729 | A4 | 11 October 2023 |
| | | | | JP | 2023089190 | A | 27 June 2023 |
| | | | | MA | 55033 | A | 29 December 2021 |
| | | | | CL | 2021002182 | A1 | 18 March 2022 |
| | | | | JOP | 20210229 | A1 | 30 January 2023 |
| | | | | AU | 2020225202 | A1 | 12 August 2021 |
| | | | | AU | 2020225202 | B2 | 26 October 2023 |
| | | | | US | 2020262911 | A1 | 20 August 2020 |
| | | | | US | 11634485 | B2 | 25 April 2023 |
| | | | | CA | 3129901 | A1 | 27 August 2020 |
| | | | | ECSP | 21060917 | A | 30 September 2021 |
| | | | | DOP | 2021000170 | A | 30 September 2021 |
| | | | | JP | 2022520857 | A | 01 April 2022 |
| | | | | JP | 7266108 | B2 | 27 April 2023 |
| | | | | AU | 2023251529 | A1 | 18 January 2024 |
| | | | | KR | 20210114989 | A | 24 September 2021 |
| | | | | IL | 285134 | A | 30 September 2021 |
| CN | 109679920 | A | 26 April 2019 | None | | | |
| CN | 111032691 | A | 17 April 2020 | CA | 3073597 | A1 | 28 February 2019 |
| | | | | EP | 3672988 | A1 | 01 July 2020 |
| | | | | JP | 2020531517 | A | 05 November 2020 |
| | | | | JP | 7212675 | B2 | 25 January 2023 |
| | | | | US | 2020140537 | A1 | 07 May 2020 |
| | | | | WO | 2019040230 | A1 | 28 February 2019 |
| | | | | US | 2022275078 | A1 | 01 September 2022 |
| CN | 113631574 | A | 09 November 2021 | KR | 20210122243 | A | 08 October 2021 |
| | | | | IL | 284644 | A | 31 August 2021 |
| | | | | MA | 54857 | A | 08 December 2021 |
| | | | | SG | 11202107995 | SA | 30 August 2021 |
| | | | | EP | 3917954 | A1 | 08 December 2021 |
| | | | | WO | 2020157305 | A1 | 06 August 2020 |
| | | | | US | 2022127350 | A1 | 28 April 2022 |
| | | | | CA | 3127935 | A1 | 06 August 2020 |
| | | | | AU | 2020215795 | A1 | 29 July 2021 |

Form PCT/ISA/210 (patent family annex) (July 2022)

**INTERNATIONAL SEARCH REPORT**
Information on patent family members

International application No.

**PCT/CN2023/137845**

| Patent document cited in search report | Publication date (day/month/year) | Patent family member(s) | | Publication date (day/month/year) |
|---|---|---|---|---|
| | | JP 2022523908 A | | 27 April 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106892982 B

- CN 109796534 A

**Non-patent literature cited in the description**

- **SINGLETON et al.** Dictionary of Microbiology and Molecular Biology. J. Wiley & Sons, 1994
- **SAMBROOK et al.** Molecular Cloning, A Laboratory Manual. Cold Springs Harbor Press, 1989
- **KABAT et al.** *Ann. NY Acad. Sci.*, 1971, vol. 190, 382-93
- **KABAT et al.** Sequences of Proteins of Immunological Interest. U.S. Department of Health and Human Services, 1991

- Cell Culture. World Publishing Corporation, January 2018
- Cell Culture Technology. Chemical Industry Press, August 2007
- Tissue and Cell Culture Technology. People's Medical Publishing House, June 2014
- **R.C. ROWE et al.** Handbook of Pharmaceutical Excipients. Chemical Industry Press, 2005